Europäisches Patentamt

(19) **European Patent Office**

Office européen des brevets

(11) Numéro de publication: **0 058 584**
**B1**

(12) **FASCICULE DE BREVET EUROPEEN**

(45) Date de publication du fascicule du brevet:
24.07.85

(51) Int. Cl.⁴: **A 61 K 9/14**, A 61 K 9/48, C 01 F 5/40, C 01 F 11/30

(21) Numéro de dépôt: **82400110.1**

(22) Date de dépôt: **21.01.82**

(54) **Procédé de fabrication d'une poudre stable contenant un produit actif très hygroscopique.**

(30) Priorité: **13.02.81 FR 8102844**

(43) Date de publication de la demande:
**25.08.82 Bulletin 82/34**

(45) Mention de la délivrance du brevet:
**24.07.85 Bulletin 85/30**

(84) Etats contractants désignés:
**AT BE CH DE GB IT LI LU NL SE**

(56) Documents cités:
**DE - A - 2 512 247**
**US - A - 2 175 083**
**US - A - 2 879 161**
**US - A - 3 297 529**
**US - A - 3 613 579**

**HAGERS HANDBUCH DER PHARMAZEUTISCHEN PRAXIS, 4ième édition, 1973, vol.IV, Springer Verlag, Berlin (DE), page 910**

**Le dossier contient des informations techniques présentées postérieurement au dépôt de la demande et ne figurant pas dans le présent fascicule.**

(73) Titulaire: **Grimberg, Georges Serge, 123 rue de l'Université, F-75007 Paris (FR)**

(72) Inventeur: **Grimberg, Georges Serge, 123 rue de l'Université, F-75007 Paris (FR)**

(74) Mandataire: **Madeuf, Claude Alexandre Jean et al, CABINET MADEUF 3, avenue Bugeaud, F-75116 Paris (FR)**

## Description

Comme on le sait certains produits pharmaceutiques renferment des substances qui contiennent dans leur molécule un nombre «X» de molécules d'eau, ce qui les rend très difficiles à conserver du fait que l'on ne peut pas les inclure dans des gélules dures ou dans des sachets. Ainsi, leur vie en pot est très réduite particulièrement dans les pays à forte humidité tels que les pays tempérés ou les zones équatoriales.

On a donc jusqu'à présent utilisé des moyens mécaniques importants tels que des emballages sous vide mais le prix de revient est alors très élevé, et cette augmentation du prix de l'emballage freine la vente du médicament qui, en général, est d'un prix de revient extrêmement bas.

Ceci est particulièrement le cas lorsque le médicament contient du chlorure de calcium hydraté $Cl_2 Ca, 6H_2O$ ou du sulfate de magnésium hydraté $SO_4Mg, 7 H_2O$. En effet, ces substances sont à l'air très hygroscopiques et, si on les place sans protection spéciale dans des gélules dures, la substance se modifie et la capsule ou gélule se ramollit, en entraînant ainsi une obligation de destruction du médicament qui n'est plus ingérable.

On a donc pensé à introduire une poudre dessicative dans le produit actif contenant des molécules d'eau et, de ce fait, on a utilisé du talc ou un autre produit analogue, mais ces substances présentent deux inconvénients majeurs, à savoir l'introduction d'un minéral qui n'est pas sans action sur le pH gastrique ou intestinal et également l'absorption de la substance active sur la poudre dessicative, ce qui la rend plus ou moins disponible pour l'organisme.

On connaît également du fait du brevet américain 2 175 083 la fabrication d'une poudre stable par encapsulation de composés hygroscopiques auxquels est ajouté un anti-hygroscopique choisi dans le groupe comprenant des mono et des diglycérides d'acides gras supérieurs.

On connaît également par le brevet allemand 2 512 247 et par l'état antérieur de la technique, l'emploi de sulfates divers et de glucoses pour former des comprimés.

La présente invention remédie à ces inconvénients en créant un procédé de fabrication d'une poudre stable en partant d'un sel minéral hygroscopique cristallisé qui contient dans sa molécule de nombreuses molécules d'eau qui la rendent déliquescente et impropre à sa mise en gélules dures ou en sachets, ce procédé consistant à broyer le sel minéral hygroscopique cristallisé et dont le broyage s'effectue en présence de levure sèche finement pulvérulente ayant une très fine granulométrie et à raison de 7,7 à 25% en poids par rapport au sel jusqu'à obtention d'un mélange homogène finement pulvérulent, stable et susceptible d'être facilement mise en gélules dures ou en sachets.

Suivant une caractéristique de l'invention, on utilise 120 kg de sel minéral hygroscopique cristallisé, particulièrement le chlorure de calcium hydraté $Cl_2Ca, 6 H_2O$ ou le sulfate de magnésium hydraté $SO_4 Mg, 7 H_2O$ que l'on broie finement en présence de 10 à 40 kg de levure sèche d'une très fine granulométrie.

Diverses caractéristiques de l'invention ressortent d'ailleurs de la description détaillée qui suit. Comme il a été expliqué ci-dessus, l'invention consiste en un procédé dans lequel on réduit en poudre fine le sel minéral hygroscopique cristallisé en présence d'une poudre sèche et finement pulvérisée anhydre de levure sèche.

La levure sèche est une matière organique alimentaire, riche en vitamines et en produits biologiques, bienfaisante pour l'organisme.

Exemples non limitatifs du procédé
de fabrication
1° 120 kg de $SO_4$ Mg, 7 $H_2$ O cristallisés sont finement broyés en présence de 10 kg de levure sèche d'une très fine granulométrie.

Le mélange obtenu se répartit parfaitement en capsules dures et se conserve des années sous blisters ou en piluliers.
2° 120 kg de $Cl_2$ Ca, 6 $H_2O$ cristallisés sont finement broyés en présence de 40 kg de levure sèche d'une granulométrie très fine.

Le mélange obtenu se répartit parfaitement en capsules dures et se conserve des années sous blisters ou en piluliers.

Le broyage est réalisé par tout moyen convenable utilisé normalement dans la technique considérée.

## Revendications

1. Procédé de fabrication d'une poudre stable d'un sel minéral hygroscopique cristallisé contenant dans sa molécule de nombreuses molécules d'eau qui la rendent déliquescente et impropre à sa mise en gélules dures ou en sachets, caractérisé en ce qu'on broie le sel minéral hygroscopique cristallisé en présence de levure sèche ayant une très fine granulométrie et à raison de 7,7 à 25% en poids par rapport au sel, jusqu'à obtention d'un mélange homogène finement pulvérulent, stable et susceptible d'être facilement mis en gélules dures ou en sachets.

2. Procédé suivant la revendication 1, caractérisé en ce qu'on utilise 120 kg de $SO_4$ Mg, 7 $H_2O$ hygroscopique cristallisé que l'on broie finement en présence de 10 kg de levure sèche d'une très fine granulométrie.

3. Procédé suivant la revendication 1, caractérisé en ce que 120 kg de $Cl_2$ Ca, 6 $H_2O$ hygroscopique sous forme cristallisée sont finement broyés en présence de 40 kg de levure sèche d'une granulométrie très fine.

## Claims

1. Process for preparing a stable powder of a crystallized hygroscopic mineral salt containing in its molecule a large number of water molecules making it deliquescent and impossible to be distributed in hard capsules or in bags, characterized by crushing the crystallized hygroscopic mineral salt in the presence of dry yeast having a very fine particle size and within the range of 7.7 to 25% in weight with respect to the salt, up to obtaining a finely pulverulent homogeneous mixture which is stable and able to be easily put in hard capsule or in bags.

2. Process according to claim 1, characterized by using 120 kg of crystallized hygroscopic $MgSO_4$, 7 $H_2O$ which is finely crushed in the presence of 10 kg of dry yeast of a very fine particle size.

3. Process according to claim 1, characterized in that 120 kg of hygroscopic $CaCl_2$, 6 $H_2O$ in a crystallized form are finely crushed in the presence of 40 kg of dry yeast of a very fine particle size.

**Patentansprüche**

1. Verfahren zur Herstellung eines stabilen Pulvers aus einem kristallisierten hygroskopischen anorganischen Salz, das in seinem Molekül zahlreiche Wassermoleküle enthält, wodurch es zerfliessbar und ungeeignet zur Bereitung harter Gelkügelchen oder Sachets ist, dadurch gekennzeichnet, dass das kristallierte hygroskopische anorganische Salz in Anwesenheit einer trockener, gemäss Gewichtsprozentsatz von 7,7 bis 25% in bezug auf das Salz zugesetzten Hefe mit einer sehr feinen Korngrösse bis zur Bildung eines feinpulverisierten homogenen Gemisches zerrieben wird, das stabil und leicht zu harten Gelkügelchen oder Sachets bereitet werden kann.

2. Verfahren nach Anspruch 1, dadurch gekennzeichnet, dass man 120 kg von kristallinem hygroskopischen $SO_4$ Mg, 7 $H_2O$ verwendet, das in Anwesenheit von 10 kg trockener Hefe mit einer sehr feinen Korngrösse fein zerrieben wird.

3. Verfahren nach Anspruch 1, dadurch gekennzeichnet, dass 120 kg von kristallinem hygroskopischen $Cl_2$ Ca, 6 $H_2O$ in Anwesenheit von 40 kg trockener Hefe mit einer sehr feinen Korngrösse fein zerrieben werden.